# EUROPÄISCHE PATENTSCHRIFT

(11) **EP 0 649 639 B1**
(45) Veröffentlichungstag und Bekanntmachung des Hinweises auf die Patenterteilung: **19.08.1998**
(21) Anmeldenummer: 93810736.4
(22) Anmeldetag: 20.10.1993
(51) Int. Cl.: A61F 2/30, A61F 2/36

(54) **Endoprothesenschaft, insbesondere einer Femurschaftprothese, mit zugeordnetem Implantat**
Endoprosthetic shaft, especially of a femoral shaft prosthesis, with associated implant
Tige endoprothétique, en particulier d'une prothèse de tige fémorale, avec implant associé

(43) Veröffentlichungstag der Anmeldung: 26.04.1995
(73) Patentinhaber: Sulzer Orthopädie AG, 6340 Baar (CH)
(72) Erfinder: Wagner, Heinz, Prof. Dr.-med., D-90592 Schwarzenbruck (DE); Willi, Roland, CH-8413 Neftenbach (CH)
(74) Vertreter: Trieblnig, Adolf

(56) Entgegenhaltungen:
- CH-A- 659 580
- DE-A- 3 132 543
- DE-U- 8 408 893
- FR-A- 2 610 824
- US-A- 4 728 335

## Beschreibung

Die Erfindung betrifft ein einem Schaft einer Endoprothese, insbesondere einer Femurkopfprothese, zugeordnetes Implantat als Ersatz für eine fehlende Knochenpartie, insbesondere den grossen Trochanter, mit einem dieser Knochenpartie entsprechend geformten, seitlich des Schafts positionierbaren Ansatzkörper, der mit Oeffnungen zum Befestigen von an ihm angreifenden Muskeln oder Bändern versehen und mit einer auf einem Längenabschnitt des Schafts anbringbaren, diesen umgebenden Hülse verbunden ist.

Ferner betrifft die Erfindung eine mit einem solchen Implantat versehene Endoprothese.

Aus der CH-PS 659 580 ist ein den grossen Trochanter ersetzendes Implantat der genannten Art bekannt, welches einen gegenüber dem Schaft einer Femurkopfprothese lateral versetzt angeordneten Ansatzkörper und eine damit zu einer Baueinheit vereinigte, auf den Schaft aufsetzbare Hülse enthält. Das bekannte Implantat ist zur Verwendung an einer Reoperationsprothese vorgesehen, die als einzementierte Zweit- oder Drittprothese bei Patienten Verwendung findet, bei denen eine früher eingesetzte Endoprothese, etwa wegen Lockerung des Prothesenschafts, reoperiert und ersetzt werden muss und bei denen - etwa in Folge von Abbauerscheinungen und Demineralisation des Knochenmaterials - der als Muskelansatzkörper dienende grosse Trochanter fehlt. Der bekannte Ansatzteil ist mit einer Vielzahl Bohrungen versehen, die zum Annähen von Muskelenden und Bändern am Ansatzteil dienen. Das aus dem Ansatzkörper und der Hülse bestehende Implantat wird mit der Hülse in axialer Richtung auf den in den Femurknochen einzusetzenden Schaft aufgeschoben und an diesem relativ zu dem auf eine Halspartie des Schafts aufzusetzenden Kopf der Prothese in einer vorgegebenen Höhe durch Anschrauben oder mittels einer Löt- oder Schweissverbindung befestigt. Das bekannte Implantat muss somit vor dem Einsetzen bzw. vor dem Einzementieren des Schafts in den Femurknochen in der den jeweiligen anatomischen Verhältnissen entsprechenden Stellung mit dem Schaft fest verbunden werden. Entsprechend ist eine nachträgliche Korrektur der Höhenlage des Implantats auf dem eingesetzten Schaft nicht möglich.

Der Erfindung liegt die Aufgabe zugrunde, ein insbesondere in dieser Hinsicht weiter entwickeltes, verbessertes Implantat zu schaffen, welches mit unterschiedlichen, z.B. millimeterweise abgestuften Abmessungen ausgeführt werden kann und welches aus einem bereitgestellten Vorrat solcher unterschiedlicher Ausführungen ausgewählt und nach dem Einsetzen des Schafts in das Knochenbett in einer den gegebenen anatomischen Verhältnissen entsprechenden Position am Schaft angebracht oder ausgewechselt werden kann, ohne den Schaft aus dem ihn umgebenden Knochenbett zu lösen.

Diese Aufgabe wird gemäss der Erfindung dadurch gelöst, dass zumindest die Hülse in zwei je für sich an den Schaft beidseitig ansetzbare und miteinander verbindbare Teile unterteilt ist, welche mit aneinander anlegbaren Stützflächen ausgeführt und über lösbare Befestigungsmittel gegeneinander verspannbar sind.

Das erfindungsgemäss ausgebildete Implantat ist für im Knochen einzementierte oder zementfrei verspannt gehaltene Prothesenschäfte verwendbar, unabhängig von der jeweiligen Befestigungsart positionierbar und gestattet auf einfache Weise ein Anbringen und Abnehmen bzw. Auswechseln des die fehlende Knochenpartie ersetzenden Ansatzkörpers. Entsprechend ist auch während einer relativ späten Phase des Implantationsvorganges - bei bereits definitiv positioniertem Schaftkörper - noch eine allfällig erforderliche Anpassung der Form der Endoprothese an die jeweils gegebenen anatomischen Verhältnisse im Implantationsbereich möglich. Die erfindungsgemässe Ausführung gestattet ferner eine den unterschiedlichen Funktionen des Ansatzkörpers und der Hülse entsprechende, von der Ausbildung der Hülse unabhängige Formgebung des Ansatzkörpers, so dass eine gegenüber bisherigen Ausführungen verbesserte Zugänglichkeit des Ansatzkörpers zum Befestigen der Muskeln und/oder Bänder erzielbar ist. Ein weiterer Vorteil des erfindungsgemäss ausgebildeten Implantats besteht darin, dass der damit zu bestückende Prothesenschaft in einer für das Einsetzen in den Implantationsbereich vorteilhaft schlanken Bauform ausgeführt werden kann, welche wenig Platz beansprucht. Der Schaft kann ferner frei von Befestigungselementen, insbesondere ohne Kerben von Befestigungsgewinden oder dgl., ausgeführt werden, so dass die im Schaft auftretenden Maximalspannungen entsprechend klein gehalten werden können.

In den abhängigen Patentansprüchen sind vorteilhafte Ausgestaltungen des Erfindungsgegenstandes angegeben.

Weitere Merkmale und Einzelheiten ergeben sich aus der folgenden Beschreibung eines in der Zeichnung schematisch dargestellten Ausführungsbeispiels der Erfindung, in Verbindung mit den Patentansprüchen. In der Zeichnung zeigen:
- Fig. 1: eine mit einem erfindungsgemäss ausgebildeten Implantat versehene Endoprothese in einer Teilansicht mit Teilschnitt;
- Fig. 2: eine Teilansicht der Endoprothese in Richtung des Pfeils II in Fig. 1;
- Fig. 3: eine Teilansicht mit Teilschnitt der Endoprothese entsprechend der Linie III-III in Fig. 1 und
- Fig. 4: das Implantat nach Fig. 1 in einer Ansicht gemäss Pfeil IV in Fig. 3.

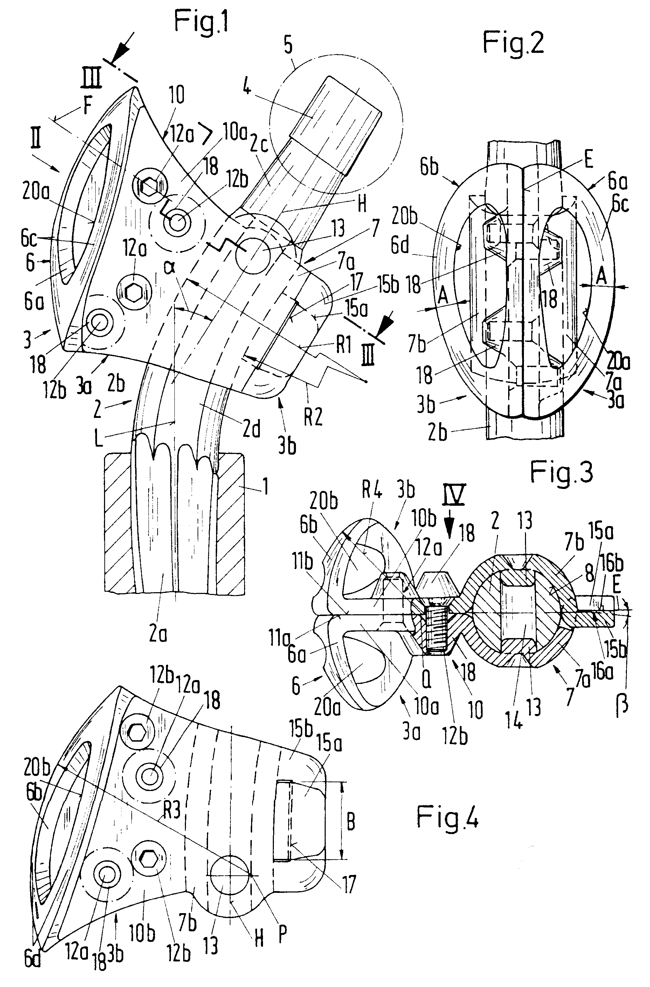

Die Endoprothese nach Fig. 1, darstellungsgemäss eine Femurkopfprothese, ist Teil einer Reoperationsprothese, die als Zweit- oder Dritt-Prothese zur Verwendung bei Patienten vorgesehen ist, bei denen eine entsprechende, früher eingesetzte Endoprothese aus irgendwelchen Gründen, z.B. wegen Lockerung ihrer Verankerung im Knochenmaterial, reoperiert und ersetzt werden muss. Die dargestellte Femurkopfprothese enthält einen in einen Femurknochen 1 implantierbaren und in diesem mittels Knochenzement oder, wie dargestellt, zementfrei fixierbaren Schaft 2 sowie ein an diesem zusätzlich anbringbares Implantat 3 als Ersatz für eine fehlende Knochenpartie, beim dargestellten Beispiel den grossen Trochanter des Femurknochens 1. Der grosse Trochanter muss, insbesondere in Reoperationsfällen, etwa in Folge von Abbauerscheinungen und Demineralisation des Knochengewebes häufig teilweise oder, wie dargestellt, vollständig reseziert werden. Der Schaft 2 ist mit einem bezüglich seiner Längsachse L konisch sich verjüngenden, in das Knochengewebe einführbaren Schaftkörper 2a ausgeführt, der in seinem proximalen Bereich über einen gekrümmten Längenabschnitt 2b in eine Halspartie 2c übergeht, deren Längsachse H unter einem Winkel α zur Längsachse L geneigt ist. Die Halspartie 2c endet in einem Konus 4, auf den ein strichpunktiert dargestellter künstlicher Gelenkkopf 5 aufsetzbar ist.

Das Implantat 3 ist mit einem gegenüber dem proximalen Bereich des Schafts 2 lateral versetzt positionierbaren Ansatzkörper 6 für daran zu befestigende, nicht dargestellte Muskeln und Bänder und einer auf dem Längenabschnitt 2b befestigbaren Hülse 7 ausgeführt. Die Hülse 7 weist einen Hohlraum 8 auf, der an den beim dargestellten Beispiel zylindrischen, mit ebenen seitlichen Führungsflächen 2d versehenen Längenabschnitt 2b angepasst und mit einer dem Verlauf des Längenabschnitts 2b entsprechenden Krümmung ausgeführt ist, die durch einen äusseren Krümmungsradius R1 und einen inneren Krümmungsradius R2 bestimmt ist. Der Ansatzkörper 6 ist in Form eines im Abstand vom Längenabschnitt 2b angeordneten, flanschartigen Schildes ausgeführt, der über eine an ihm stegartig anschliessene Tragpartie 10 mit der Hülse 7 verbunden und durch diese relativ zum Mittelpunkt des Gelenkkopfs 5 in einer vorbestimmten Höhenlage gehalten ist, um für die Befestigung der Muskeln und Bänder eine den natürlichen anatomischen Verhältnissen möglichst ähnliche Anordnung zu erhalten.

Wie insbesondere aus den Fig. 2 und 3 hervorgeht, sind der Ansatzkörper 6 und die Tragpartie 10 sowie die Hülse 7 je in zwei aneinander anlegbare und voneinander trennbare Ansatzteile 6a und 6b bzw. Stegteile 10a und 10b bzw. Hülsentelle 7a und 7b unterteilt, wobei die Teile 6a, 10a und 7a zu einer Baueinheit 3a, und die Teile 6b, 10b und 7b zu einer Baueinheit 3b vereinigt sind. Die Baueinheiten 3a und 3b bilden zwei je für sich an den Schaft 2 beidseitig ansetzbare Teile des Implantats 3, welche mit aneinander anlegbaren Stützflächen 11a und 11b ausgeführt und über lösbare Befestigungsmittel, darstellungsgemäss Schrauben 12a und 12b, gegeneinander und gegen den Schaft 2 verspannbar sind. Die Stützflächen 11a und 11b können darstellungsgemäss in einer durch die Längsachsen L und H bestimmten Ebene E zusammenwirken. Zur Sicherung der Einbaulage des Implantats 3 kann mindestens einer oder, wie dargestellt, jeder der Hülsenteile 7a und 7b mit einem nach innen in den Hohlraum 8 vorstehenden, zapfenartigen Zentrierteil 13 versehen sein, der zum Einführen in eine entsprechende, am Schaft 2 ausgebildete Aufnahmebohrung 14 bestimmt ist.

Die Hülsenteile 7a und 7b sind an der dem Ansatzkörper 6 abgewandten Seite mit ineinander verschränkbaren, in Richtung der Ebene E abgebogenen Endpartien 15a und 15b versehen, welche mit im Bereich der Ebene E aneinander anlegbaren Stützflächen 16a bzw. 16b ausgeführt sind. Darstellungsgemäss kann die Endpartie 15a mit einer gegenüber dem Hülsenteil 7a reduzierten Breite B ausgeführt sein, wobei die Endpartie 15b mit einer entsprechenden Durchstecköffnung 17 für die Endpartie 15a versehen ist und die Stützflächen 16a und 16b je durch eine Fortsetzung der Aussenseite des betreffenden Hülsenteils 7a bzw. 7b gebildet sind. Die Endpartie 15a kann, wie in Fig. 3 strichpunktiert angedeutet, so gebogen sein, dass ihre Stützfläche 16a mit der Ebene E einen Winkel β von ca. 0° bis 5° einschliesst, der eine gegenseitige elastische Verspannung der Endpartien 15a und 15b ermöglicht. Die Stegteile 10a und 10b sind je mit Durchtrittsbohrungen für die sie durchsetzenden Schrauben 12a bzw. 12b sowie mit in örtlichen Verdickungen 18 angeordneten Gewindebohrungen zur Aufnahme der durch den jeweils anderen Stegteil 10b bzw. 10a einführbaren Schrauben 12b bzw. 12a versehen.

Der durch die Ansatzteile 6a und 6b gebildete Ansatzkörper 6 ist in Form eines im Abstand vom Längenabschnitt 2b des Schafts 2 verlaufenden Schildes ausgeführt, welcher entsprechend der Darstellung nach Fig. 2 eine im wesentlichen ovale Aussenkontur aufweist und welcher mit einer gewölbten Ansatzfläche für die daran zu befestigenden Muskeln und/oder Bänder ausgeführt ist, die eine entlang der Längserstreckung des Schafts 2 bzw. des Längenabschnitts 2b verlaufende Krümmung in Längsrichtung und eine diese überlagernde Krümmung in Querrichtung aufweist. Die Ansatzfläche ist durch bezüglich der Ebene E symmetrische Teilflächen 6c und 6d gebildet, deren Krümmung in Längsrichtung je im Bereich der Ebene E durch einen ersten Krümmungsradius R3 bestimmt ist und deren Krümmungen in Querrichtung je durch einen zweiten Krümmungsradius R4 bestimmt sind, der kleiner ist als der erste Krümmungsradius R3. Wie aus den Fig. 1 und 4 hervorgeht, ist der erste Krümmungsradius R3 zumindest annähernd auf einen Punkt P im Bereich der Längsachse H des Schafts 2, darstellungsgemäss nahe der Aufnahmebohrung 14, zentriert, während der zweite Krümmungsradius R4 gemäss Fig. 3 auf einen Punkt Q zentriert ist, der mit dem Punkt P in einer zur Längsachse H im wesentlichen senkrechten Ebene F liegen kann.

Auf diese Weise kann eine den natürlichen Gegebenenheiten weitgehend entsprechende, an unterschiedliche anatomische Verhältnisse anpassbare Auflage für die normalerweise am grossen Trochanter zu befestigenden Muskeln und/oder Bänder erzielt werden. Der Ansatzkörper 6 kann mit mehreren, darstellungsgemäss zwei, je eine der Ansatzteile 6a bzw. 6b durchsetzenden Durchbrüchen 20a und 20b ausgeführt sein, welche sich, der Aussenkontur der Ansatzteile 6a und 6b folgend, je über einen zusammenhängenden inneren Bereich der Ansatzfläche 6c bzw. 6d erstrecken. Entsprechend können die Durchbrüche 20a und 20b, wie dargestellt, nach aussen durch rahmenartige Umfangspartien begrenzt sein, welche je mit im wesentlichen konstanter Breite A ausgeführt sind und welche dadurch eine allseitig gut zugängliche Auflage für die daran zu befestigenden Muskeln und/oder Bänder bilden.

Die miteinander zu verbindenden Teile des erfindungsgemäss ausgebildeten Implantats 3 können auf einfache Weise, von einer für den Operateur gut zugänglichen Seite her, in den Implantationsbereich eingeführt, hinter dem Schaft 2 mit den Endpartien 15a und 15b zusammengeführt, ineinander verhakt und anschliessend über dem Schaft 2 geschlossen und mittels der Schrauben 12a und 12b miteinander verbunden werden. Die Anbringung bzw. ein allfälliges Auswechseln des Implantats 3 auf dem Schaft 2 erfordert somit keine speziellen, in den Rückraum hinter dem Schaft 2 einführbaren Verbindungselemente und Werkzeuge, sowie - ausser dem Zusammenführen der Endpartien 15a und 15b - keine in diesem Rückraum vorzunehmenden Manipulationen. Mit dem erfindungsgemässen Implantat ist daher eine wesentliche Arbeits- und Zeitersparnis erzielbar. Die Teile des Implantats 3 können aus einem körperverträglichen Metall, z.B. Titan, oder einem entsprechend geeigneten Kunststoff gefertigt sein.

Entsprechend abgewandelte Ausführungsformen des Implantats sind auch für Anwendungen ausserhalb des Femurbereichs, z.B. im Bereich des Schultergelenks, geeignet.

Nach einer anderen, nicht dargestellten Ausführungsform kann der Ansatzkörper 6 - oder ein entsprechender Teil eines abgewandelten Implantats - einteilig ausgeführt und lediglich die Hülse 7 in zwei beidseitig an den Schaft 2 anlegbare und miteinander verbindbare Hülsenteile 7a, 7b unterteilt sein, von denen nur einer mit dem einteiligen Ansatzkörper verbunden ist. Es ist auch eine Ausführung möglich, bei der die Hülse 7 in Hülsenteile unterteilt ist, welche über quer zur Ebene E gestellte Stützflächen zusammenwirken, die z.B. in einer Ebene liegen, die in Fig. 1 senkrecht zur Zeichenebene durch die Längsachse H verläuft.

Zusammenfassend lässt sich die Erfindung wie folgt beschreiben: Das Implantat 3 enthält, als Ersatz für eine fehlende Knochenpartie, insbesondere den grossen Trochanter, einen seitlich des Schafts 2 der zugehörigen Prothese positionierbaren Ansatzkörper 6 in Form eines flanschartigen Schildes für daran zu befestigende Muskeln und Bänder, sowie eine am Schaft 2 anbringbare Hülse 7, welche in zwei beidseitig an den Schaft 2 ansetzbare und miteinander verbindbare Hülsenteile 7a und 7b unterteilt ist. Der Ansatzkörper 6 ist ebenfalls in zwei aneinander anlegbare Ansatzteile 6a und 6b unterteilt, welche je mit einem der Hülsenteile 7a bzw. 7b zu einer Baueinheit 3a bzw. 3b vereinigt und über zwischen dem Schaft 2 und den Ansatzteilen 6a und 6b angeordnete Schrauben 12a und 12b miteinander lösbar verbunden sind. Das Implantat 3 ist von einer dem Operateur gut zugänglichen Seite her auf dem Schaft 2 anbringbar bzw. auf entsprechend einfache Weise auswechselbar.

## Patentansprüche

1. Einem Schaft (2) einer Endoprothese, insbesondere einer Femurkopfprothese, zugeordnetes Implantat (3) als Ersatz für eine fehlende Knochenpartie, insbesondere den grossen Trochanter, mit einem dieser Knochenpartie entsprechend geformten, seitlich des Schafts (2) positionierbaren Ansatzkörper (6), der mit Oeffnungen zum Befestigen von an ihm angreifenden Muskeln oder Bändern versehen und mit einer auf einem Längenabschnitt (2b) des Schafts (2) anbringbaren, diesen umgebenden Hülse (7) verbunden ist, dadurch gekennzeichnet, dass zumindest die Hülse (7) in zwei je für sich an den Schaft (2) beidseitig ansetzbare und miteinander verbindbare Teile (7a und 7b) unterteilt ist, welche mit aneinander anlegbaren Stützflächen (16a, 16b) ausgeführt und über lösbare Befestigungsmittel (12a, 12b) gegeneinander verspannbar sind.

2. Implantat nach Anspruch 1, dadurch gekennzeichnet, dass der Ansatzkörper (6) in Form eines im Abstand vom Schaft (2) angeordneten, flanschartigen Schildes ausgeführt und über eine an diesen anschliessende, stegartige Tragpartie (10) mit mindestens einem der Hülsenteile (7a, 7b) verbunden ist.

3. Implantat nach Anspruch 1 oder 2, dadurch gekennzeichnet, dass der Ansatzkörper (6) in zwei aneinander anlegbare und voneinander trennbare Ansatzteile (6a und 6b) unterteilt ist, welche je mit einem der Hülsenteile (7a bzw. 7b) zu einer Baueinheit (3a bzw. 3b) verbunden und durch die Befestigungsmittel (12a, 12b) miteinander koppelbar sind.

4. Implantat nach Anspruch 3, dadurch gekennzeichnet, dass die Ansatzteile (6a und 6b) über Stützflächen (11a und 11b) zusammenwirken, die mit den Stützflächen (16a und 16b) der Hülsenteile (7a und 7b) in einer gemeinsamen, z.B. bezüglich einer Längsachse (L, H) des Schafts (12) ausrichtbaren Ebene (E) liegen.

5. Implantat nach einem der Ansprüche 2 bis 4, dadurch gekennzeichnet, dass der Ansatzkörper (6) mit mindestens zwei beidseits der Tragpartie (10) angeordneten Durchbrüchen (20a, 20b) ausgeführt ist, die sich je über einen zusammenhängenden inneren Flächenbereich des Schildes erstrecken und die nach aussen durch rahmenartige Umfangsbereiche des Schildes begrenzt sind.

6. Implantat nach einem der Ansprüche 2 oder 5, dadurch gekennzeichnet, dass der Schild mit einer gewölbten Ansatzfläche (6c, 6d) für die Muskeln und Bänder ausgeführt ist, die durch eine entlang der Längserstreckung des Schafts (2) verlaufende Krümmung in Längsrichtung und eine diese überlagernde Krümmung in Querrichtung gebildet ist.

7. Implantat nach Anspruch 6, dadurch gekennzeichnet, dass die Krümmung der Ansatzfläche (6c, 6d) in Längsrichtung durch einen ersten Krümmungsradius (R3) bestimmt ist, der zumindest annähernd auf einen Punkt (P) im Bereich der Längsachse (H) des Schafts (2) zentriert ist und dass die Krümmung in Querrichtung durch einen zweiten Krümmungsradius (R4) bestimmt ist, der kleiner ist als der erste Krümmungsradius (R3).

8. Implantat nach einem der Ansprüche 2 bis 7, dadurch gekennzeichnet, dass der Schild mit einer ovalen Aussenkontur ausgeführt ist.

9. Implantat nach einem der vorangehenden Ansprüche, dadurch gekennzeichnet, dass die Hülsenteile (7a und 7b) auf der dem Ansatzkörper (6) abgewandten Seite mit ineinander verschränkbaren und gegeneinander verspannbaren Endpartien (15a und 15b) ausgeführt und auf der dem Ansatzkörper (6) zugewandten Seite mit Mitteln (18) zum Anbringen der Befestigungsmittel (12a, 12b) versehen sind.

10. Implantat nach einem der vorangehenden Ansprüche, dadurch gekennzeichnet, dass mindestens einer der Hülsenteile (7a bzw. 7b) mit einem in seinen Hohlraum (8) vorstehenden, zum Einführen in eine Aufnahmebohrung (14) des Schafts (2) bestimmten zapfenartigen Zentrierteil (13) ausgeführt ist.

11. Endoprothese mit einem Implantat nach einem der Ansprüche 1 bis 10.

12. Endoprothese nach Anspruch 11, deren Schaft (2) einen in Richtung einer Längsachse (L) in einen Röhrenknochen einführbaren Schaftkörper (2a) und eine zur Längsachse (L) unter einem Winkel (α) geneigte Halspartie (2c) aufweist, dadurch gekennzeichnet, dass der Schaftkörper (2a) in seinem zur Aufnahme des Implantats (3) bestimmten proximalen Bereich mit einem gekrümmten Längenabschnitt (2b) ausgeführt ist, der in die Halspartie (2c) übergeht.

## Claims

1. An implant (3) associated with a shaft (2) of an endoprosthesis, in particular of a prosthesis for the head of a femur, as a replacement for a missing bone portion, in particular the large trochanter, comprises an attachment body (6) which is formed corresponding to this bone portion, which is positionable to the side of the shaft (2), which is provided with openings for the securing of muscles and ligaments attached thereto, and which is connected to a yoke (7) mountable onto and surrounding a longitudinal section (2b) of the shaft (2), characterized in that at least the yoke (7) is subdivided into two parts (7a and 7b) which can be placed on both sides of the shaft (2), which are connectable to one another, and which are executed with support surfaces (16a, 16b) which can be brought into contact with one another and can be braced against one another via releasable fastening means (12a, 12b).

2. Implant in accordance with claim 1, characterized in that the attachment body (6) is implemented in the form of a flange-like shield disposed at a distance from the shaft (2) and connected to at least one of the yoke parts (7a, 7b) via an adjoining web-like carrier portion (10).

3. Implant in accordance with claim 1 or 2, characterized in that the attachment body (6) is subdivided into two attachment parts (6a and 6b) which can be brought into contact with each other and separated from one another, each of which is connected to a respective yoke part (7a, 7b respectively) to form a respective constructional unit (3a, 3b respectively) and can be coupled to one another via the fastening means (12a, 12b).

4. Implant in accordance with claim 3, characterized in that the attachment parts (6a and 6b) cooperate via support surfaces (11a and 11b) which lie in a common plane (E) with the support surfaces (16a and 16b) of the yoke parts (7a and 7b), for example in a common plane (E) which can be aligned with respect to a longitudinal axis (L, H) of the shaft (12).

5. Implant in accordance with one of the claims 2 to 4, characterized in that the attachment body (6) is implemented with at least two apertures (20a and 20b) which are arranged on either side of the carrier portion (10), which each extend over a continuous inner areal region of the shield, and which are bounded outwardly by frame-like peripheral regions of the shield.

6. Implant in accordance with one of the claims 2 or 5, characterized in that the shield is implemented with a domed attachment surface (6c, 6d) for the muscles and ligaments, which is formed by a curvature in the longitudinal direction extending along the longitudinal extent of the shaft (2), and a curvature in the transverse direction superimposed thereon.

7. Implant in accordance with claim 6, characterized in that the curvature of the attachment surface (6c, 6d) in the longitudinal direction is specified by a first radius of curvature (R3), which is, at least approximately, centred on a point (P) in the region of the longitudinal axis (H) of the shaft (2), and in that the curvature in the transverse direction is specified by a second radius of curvature (R4) which is smaller than the first radius of curvature (R3).

8. Implant in accordance with one of the claims 2 to 7, characterized in that the shield is formed with an oval outer contour.

9. Implant in accordance with on of the preceding claims, characterized in that the yoke parts (7a and 7b) on the side remote from the attachment body (6) are provided with interlockable end portions (15a and 15b), which can be braced against one another, and are provided with means (18) for attachment of the fastening means (12a, 12b) on the side adjacent to the attachment body (6).

10. Implant in accordance with one of the preceding claims, characterized in that at least one of the yoke parts (7a and 7b) is implemented with a spigot-like centring part (13) projecting inwardly into its hollow cavity (8) and provided for insertion into a receiving bore (14) of the shaft (2).

11. Endoprosthesis with an implant in accordance with one of the claims 1 to 10.

12. Endoprosthesis in accordance with claim 11, the shaft (2) of which comprises a shaft body (2a) insertable in the direction of a longitudinal axis (L) into a tubular bone, and a neck portion (2c) which is inclined at an angle (α) relative to the longitudinal axis (L), characterized in that the shaft body (2a) is implemented in its proximal region, which is provided for the receipt of the implant (3), with a curved longitudinal section (2b) which merges into the neck portion (2c).

## Revendications

1. Implant (3) associé à une tige (2) d'une endoprothèse, notamment d'une prothèse de tête du fémur pour remplacer une partie osseuse manquante, notamment le grand trochanter, avec un corps rapporté (6), configuré de manière correspondante à cette partie osseuse, pouvant être positionné sur le côté de la tige (3) qui est pourvu d'ouvertures pour la fixation de muscles ou de bandes s'appliquant à celui-ci et qui est relié à une douille (7) applicable à un tronçon longitudinal (2b) de la tige (2), entourant celle-ci, caractérisé en ce qu'au moins la douille (7) est divisée en deux parties (7a et 7b) applicables chacune individuellement des deux côtés à la tige (2) et pouvant être reliées l'une à l'autre, qui sont réalisées avec des surfaces d'appui (16a, 16b) applicables l'une contre l'autre et qui peuvent être serrées l'une contre l'autre par des moyens de fixation amovibles (12a, 12b).

2. Implant selon la revendication 1, caractérisé en ce que le corps rapporté (6) est réalisé sous la forme d'un bouclier d'un type de bride, disposé à une certaine distance de la tige (2) et est relié par une partie de support en forme de nervure (10), faisant suite à celui-ci, à au moins l'une des parties de douille (7a, 7b).

3. Implant selon la revendication 1 ou 2, caractérisé en ce que le corps rapporté (6) est divisé en deux parties rapportées (6a et 6b) applicables l'une à l'autre et séparables l'une de l'autre, qui sont reliées respectivement à l'une des parties de douille (7a respectivement 7b) pour former une unité constructive (3a respectivement 3b) et qui peuvent être accouplées l'une avec l'autre par des moyens de fixation (12a, 12b).

4. Implant selon la revendication 3, caractérisé en ce que les parties rapportées (6a et 6b) coopèrent par des surfaces d'appui (11a et 11b) qui se situent, avec les surfaces d'appui (16a et 16b) des parties de douille (7a et 7b), dans un plan commun (E) orientable par exemple relativement à un axe longitudinal (L, H) de la tige (12).

5. Implant selon l'une des revendications 2 à 4, caractérisé en ce que le corps rapporté (6) est réalisé avec au moins deux perçages (20a, 20b) disposés de part et d'autre de la partie de support (10), qui s'étendent respectivement sur une zone de surface intérieure contigué du bouclier et qui sont délimités vers l'extérieur par des zones de pourtour en forme de cadre du bouclier.

6. Implant selon l'une des revendications 2 ou 5, caractérisé en ce que le bouclier est réalisé avec une surface rapportée courbée (6c, 6d) pour les muscles et bandes, qui est formée par une courbure s'étendant le long de l'extension longitudinale de la tige (2) dans la direction longitudinale et une courbure superposée à celle-ci dans la direction transversale.

7. Implant selon la revendication 6, caractérisé en ce que la courbure de la surface rapportée (6c, 6d) est définie dans la direction longitudinale par un premier rayon de courbure (R3) qui est centré au moins approximativement sur un point (P) au voisinage de l'axe longitudinal (H) de la tige (2), et en ce que la courbure dans la direction transversale est définie par un deuxième rayon de courbure (R4) qui est plus petit que le premier rayon de courbure (R3).

8. Implant selon l'une des revendications 2 à 7, caractérisé en ce que le bouclier est réalisé avec un contour extérieur ovale.

9. Implant selon l'une des revendications précédentes, caractérisé en ce que les parties de douille (7a et 7b) sont réalisées sur le côté éloigné du corps rapporté (6) avec des parties d'extrémité (15a et 15b) pouvant s'entrelacer et se serrer l'une contre l'autre et sont pourvues, sur le côté orienté vers le corps rapporté (6), de moyens (18) pour appliquer les moyens de fixation (12a, 12b).

10. Implant selon l'une des revendications précédentes, caractérisé en ce qu'au moins l'une des parties de douille (7a respectivement 7b) est réalisée avec une partie de centrage (13) en forme d'ergot faisant saillie dans son espace creux (8), prévu pour l'insertion dans un perçage de réception (14) de la tige (2).

11. Endoprothèse avec un implant selon l'une des revendications 1 à 10.

12. Endoprothèse selon la revendication 11, dont la tige (2) présente un corps de tige (2a) insérable dans la direction d'un axe longitudinal (L) dans un os long et une partie de col (2c) inclinée relativement à l'axe longitudinal (L) suivant un angle (α), caractérisée en ce que le corps de tige (2a) est réalisé, dans sa zone proximale prévue pour la réception de l'implant (3), avec un troncon longitudinal courbé (2b) qui rejoint la partie de col (2c).
